(19) Europäisches Patentamt
European Patent Office
Office européen des brevets

(11) Publication number: **0 135 934**
**A2**

(12) **EUROPEAN PATENT APPLICATION**

(21) Application number: **84111489.5**

(22) Date of filing: **26.09.84**

(51) Int. Cl.⁴: **C 07 C 103/365,** C 07 C 103/375, C 07 D 249/08, A 01 N 37/42

(30) Priority: **28.09.83 HU 336383**

(43) Date of publication of application: **03.04.85 Bulletin 85/14**

(84) Designated Contracting States: **CH DE FR GB LI**

(71) Applicant: **NITROKEMIA IPARTELEPEK, Pf. 45, H-8184 Füzfögyártelep (HU)**

(72) Inventor: **Dukai, József, Dr., 16, Buzavirág u., H-8200 Veszprém (HU)**
Inventor: **Horvath, András, 10, Gagarin u., H-8184 Balatonfüzfögyártelep (HU)**
Inventor: **Henger, Károly, 28, Gagarin u., H-8184 Balatonfüzfögyártelep (HU)**
Inventor: **Balogh, Marianna, Dr., 29, Egry J. u., H-8200 Veszprém (HU)**
Inventor: **Fodor, Ferenc, 13, Forrás u., H-8184 Balatonfüzfögyártelep (HU)**

(74) Representative: **von Füner, Alexander, Dr. et al, Patentanwälte Schiff, von Füner Strehl, Schübel-Hopf, Ebbinghaus, Finck Mariahilfplatz 2 u. 3, D-8000 München 90 (DE)**

(54) **New glyoxanilide derivatives, microbicidal agents and process for the preparation of the active substance.**

(57) The invention relates to glyoxanilide derivatives of the general formula

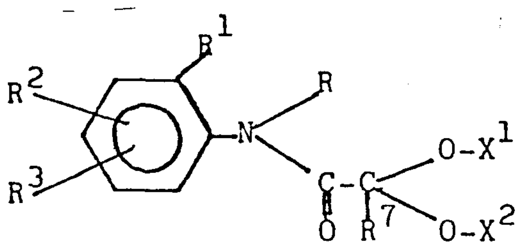

I

wherein

R stands for H, $C_1$–$C_4$ alkyl, $C_{1-4}$ alkoxy, $C_{1-4}$ alkyl, $C_2$–$C_5$ alkenyl, allyl, $C_{2-5}$ alkinyl, halomethyl, $-CH/R^4/COR^5$, $-CH/R^4/CN$, $-CHR^4/CHR^6/_nCOOR^5$, a heterocyclic group optionally substituted by one or more groups selected from alkyl and halogen having the general formula

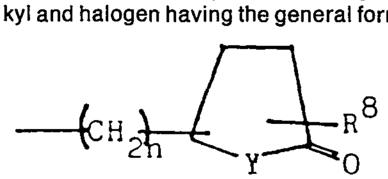

V

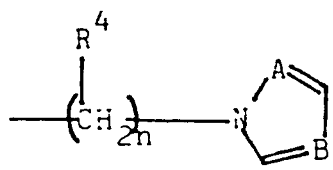

VI

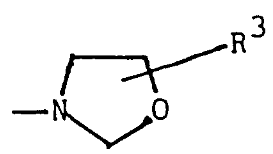

VII

or benzyl group wherein the phenyl group is optionally substitued by one or more alkyl, alkoxy, halogen, haloalkyl, nitro, nitrile or $-CH_2SCN$, $-CH_2-Y-P=Z/OR^{12}//OR^{13}/$ or an acetal group of the general formula

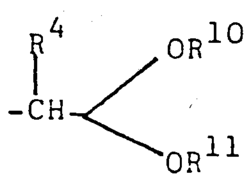

VIII

EP 0 135 934 A2

wherein

$R^1$ stands for H, $C_1$–$C_4$ alkyl, $C_1$–$C_4$ alkoxy, haloalkyl, $-COOR^{14}$,

$R^2$ stands for H, $C_1$–$C_4$ alkyl, $C_1$–$C_4$ alkoxy, halogen, haloalkyl, CN, SCN,

$R^3$ stands for H, halogen, $-CH_3$

$R^4$ represents H, $C_1$–$C_4$ alkyl

$R^5$ represents $C_1$–$C_4$ alkoxy, $-NR^{14}R^{15}$, OM

$R^6$ stands for $C_1$–$C_4$ alkyl

$R^7$ is H or $-CH_3$

$R^8$, $R^9$ represents H or $-CH_3$

$R^{10}$, $R^{11}$ stand for H, $C_1$–$C_4$ alkyl or together form a $C_1$–$C_4$ alkylene group

$R^{12}$, $R^{13}$ is H, $-CH_3$, $-C_2H_5$

$R^{14}$, $R^{15}$ represents H, $C_1$–$C_4$ alkyl or together form a $C_{4-5}$ alkylene group

$X^1$, $X^2$ stand for H, $-COR^{16}$, $C_1$–$C_4$ alkyl or together form the ethylene bridge of the 1,3-dioxolane ring

$R^{16}$ is H, $-CH_3$, $-CH_2Cl$, $-C_2H_5$

n is 0, 1, 2

Z, Y is O or S

A, B stand for $-CH=$, $-N=$

halo stands for F, Cl, Br

M is an equivalent of the following cations: Na, K, $NH_4^+$, Mg, Ca, Zn, Cu, Mn, Fe, and microbicidal agents containing the same.

# NEW GLYOXANILIDE DERIVATIVES, MICROBICIDAL AGENTS AND PROCESS FOR THE PREPARATION OF THE ACTIVE SUBSTANCE

The present invention relates to new glyoxanilide derivatives of the general formula

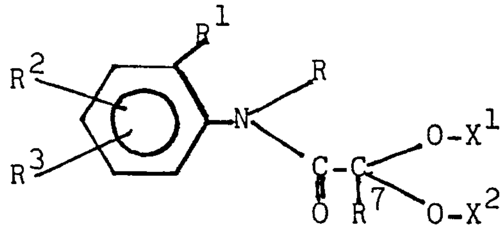

I

microbicidal agents containing the same and a process for the preparation of the new active ingredients.

Some biologically active compounds of glyoxanilide type are known. 2-Haloglyoxanilide oximes of the formula

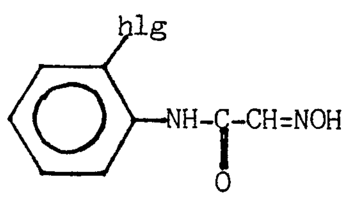

II

can be utilized as intermediates of antibiotics /Japanese Patent No. 6488'67-1965 and Chem. Abstracts 67:73322/. Glyoxanilide thio-semicarbazones of the general formula

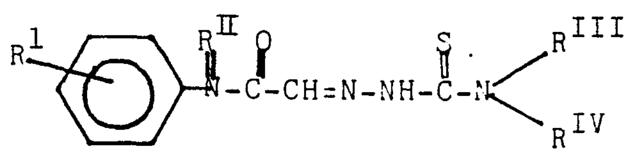

III

A3329-3073 KY

are therapeutic agents known from US-P No. 3 299 131. In GB-P Nos. 1 280 231., 1 280 232, 1280 233 among others 2'-benzoyl-/4' or 2'/-chloro-acetanilide diacetates of the formula

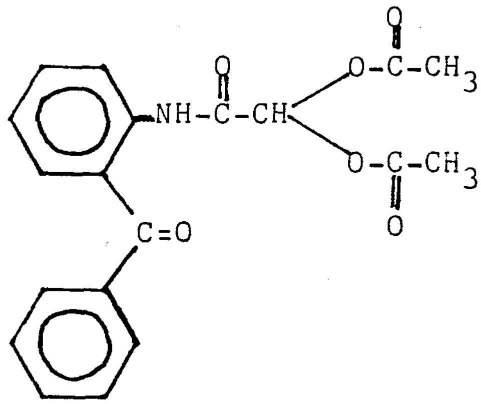

IV

are disclosed which can be used as intermediates when preparing benzodiazepine psycholeptica.

Glyoxanilides of the general formula I are new.

In the general formula I

R stands for H, $C_1$-$C_4$ alkyl, $C_{1-4}$alkoxy $C_{1-4}$ alkyl, $C_2$-$C_5$ alkenyl, allyl, $C_{2-5}$ alkinyl, halo-methyl, $-CH/R^4/COR^5$, $-CH/R^4/CN$, $-CHR^4/CHR^6/_nCOOR^5$, a heterocyclic group optionally substituted by one or more groups selected from alkyl and halogen having the general formula

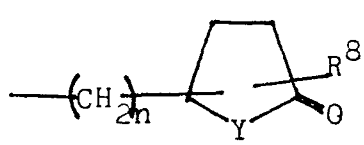

V

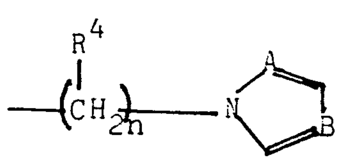

VI

0135934

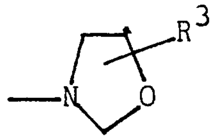

VII

or benzyl group

wherein the phenyl group is optionally substituted by one or more alkyl, alkoxy, halogen, haloalkyl, nitro, nitrile or $-CH_3SCN$, $-CH_2-Y-P{=}Z/OR^{12}/\ /OR^{13}/$ or an acetal group of the general formula

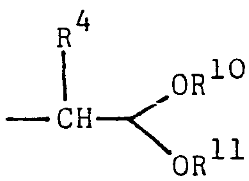

VIII

$R^1$ stands for H, $C_1$-$C_4$ alkyl, $C_1$-$C_4$ alkoxy, haloalkyl, $-COOR^{14}$,

$R^2$ stands for H, $C_1$-$C_4$ alkyl, $C_1$-$C_4$ alkoxy, halogen, haloalkyl, CN, SCN,

$R^3$ stands for H, halogen, $-CH_3$

$R^4$ represents H, $C_1$-$C_4$-alkyl

$R^5$ represents $C_1$-$C_4$ alkoxy, $-NR^{14}R^{15}$, OM

$R^6$ stands for $C_1$-$C_4$ alkyl,

$R^7$ is H or $-CH_3$

$R^8$, $R^9$ represent H or $-CH_3$

$R^{10}$, $R^{11}$ stand for H, $C_1$-$C_4$ alkyl or together form a

0135934

$C_1$-$C_4$ alkylene group,

$R^{12}$, $R^{13}$ is H, $-CH_3$, $-C_2H_5$,

$R^{14}$, $R^{15}$ represent H, $C_1$-$C_4$ alkyl or together form a $C_{4-5}$ alkylene group,

$X^1$, $X^2$ stand for H, $-COR^{16}$, $C_1$-$C_4$ alkyl or together form the ethylene bridge of the 1,3-dioxolane ring,

$R^{16}$ is H, $-CH_3$, $-CH_2Cl$, $-C_2H_5$,

n is 0, 1, 2,

Z, Y are O or S,

A, B stand for -CH=, -N=,

halo stands for F, Cl, Br,

M is an equivalent of the following cations: Na, K, $NH_4^+$, Mg, Ca, Zn, Cu, Mn, Fe.

The compounds of the general formula I can be used as active ingredients of agriculturally suitable biocidal agents. These agents usually contain 0.1 to 95 % by mass of active ingredient together with solid or liquid carriers and/or solvents, optionally with surfactants and/or other excipients, preferably with dispersing agents, wetting agents, and agents promoting adhesion and optionally other microbicidal agents.

The invention also extends to the preparation of the compounds of the general formula I according to the following variants:

a/ Aniline derivatives of the general formula

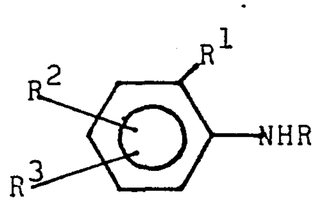

Ia

0135934

- wherein the substituents are as defined above - can be reacted with reactive derivatives, such as halides or anhydrides of glyoxylic acid acylates or acetals of the general formula

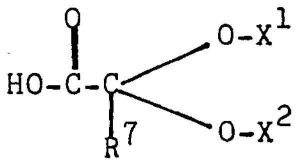

IX

in a suitable solvent, optionally in the presence of an acid binding agent at a temperature ranging from -10 to +120°C, whereafter the reaction mixture is worked up by distillation or crystallization and the desired product is isolated by washing, distillation or crystallization or

b/ azomethines of the general formula

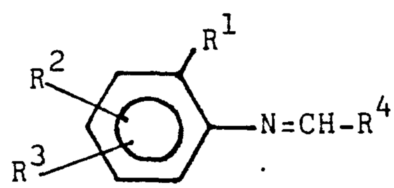

Ib

are reacted with an acid halide of glyoxylic acylate or acetal of the general formula

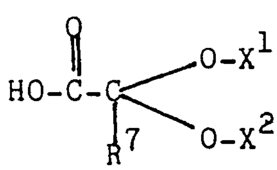

IX

in a suitable solvent at a temperature ranging from -10 to 100 $^{o}C$ whereafter the obtained intermediate product of the general formula

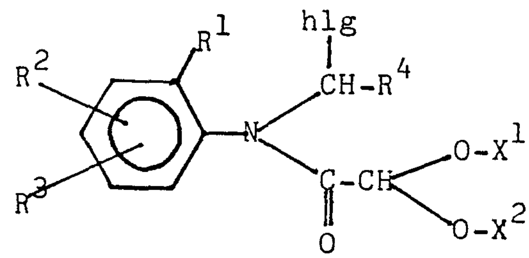

Ic

is reacted with a $C_{1-4}$ alcohol or one of the N-containing heterocyclic molecules of the general formula

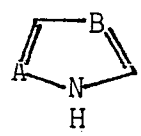

VIa

or

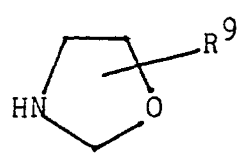

VIIa

without isolation in the presence of an acid binding agent or without it and the reaction mixture is then worked up and the microbicidal active compounds of the general formula I are isolated.

The compounds according to the invention are optionally combined with other active ingredients and worked up to solid or liquid compositions.

Solid compositions:

Dusting agents: up to 5 % active ingredient is ground together with talc or other mineral substances, such as synthetic $SiO_2$.

Granulates: Active ingredient content: 1 to 90 %. The solutions are blended with a buffer in an appropriate amount, with non-ionic and/or ionic surfactant, and are applied to a carrier /such as caolin, quartz sand, perlit/ optionally blended with film coating substance.

Water dispersible powders /WP/: active ingredient content: 5-80 %.

The powders can be diluted with water prior to use, and dispersing agent, filling agent and an agent promoting grinding and adhesion are added to the blend.

Liquid compositions:

Water dispersible emulsifiable concentrates /EC/

Active ingredient content: 5-85 %. The active ingredient is dissolved in a non-phytotoxic solvent or solvent mixture and ionic or non-ionic emulsifiers are added. The real solutions can be thus converted with water to emulsions of the desired concentration.

Suspension concentrate /FW/

The solid active ingredient is ground and its particles are dispersed in a liquid dispersion medium, in which the solubility of the active ingredient is lower than 100 ppm.

Part of the compounds according to the invention is water soluble and aqueous solutions can be prepared

from these compounds.

Part of the starting materials of the general formula Ia /R=H/ is commercially available, and those wherein R≠H can be prepared by methods known from the literature. The mentioned reactive derivatives can be prepared from the compounds of the general formula IX by method known per se, by using halogenating agents, such as thionyl halides, phosphorus halides. The anhydrides are also prepared by method known per se.

In process variant a/ for acylation water and inert solvents may be used as reaction medium. Among acid binding agents alkali hydroxides, carbonates, tertiary amines can be mentioned binding the formed hydrogen halides or compounds of the general formula IX and which can be washed at the end of the reaction.

Acylates of the general formula IX are the O-acylated derivatives of the glyoxylic hydrate form and acetales are the O-alkylated derivatives. The derivatives derive from acetic acid and can be called as 2,2-dialkoxy respectively 2,2-diacyloxy acetic acid.

For acylation reactions anilines of the general formula Ia and reactive intermediate products of glyoxylic acid aclyates, respectively acetals of the general formula IX can be used at a molar ratio of 1:1-10. The amount of the optionally used acid binding agents depends on the amount of the reactive derivative of the formula IX. The reaction temperature may vary between -10 to 120 °C, preferably 0 to 30 °C. The acylation can be performed by adding

the compound of the general formula Ia to a reactive acylating agent of the general formula IX or one may proceed the other way round.

The reaction product is usually obtained in the form of a solution and the solvent is then evaporated after washing with water, alkali, acid and again with water. The residue can be used directly or can be subjected to further purification /recrystallization, vacuum distillation/.

According to process variant b/ imines of the general formula Ib /Schiff-bases, known compounds/ are reacted with halides of glyoxylic acid acylates and acetals, respectively of the general formula IX in a suitable solvent at a temperature of -10 to +100 °C, preferably 0 to 40 °C. The obtained compounds of the general formula Ic are reacted with a suitable $C_{1-4}$ alcohol and nitrogen containing heterocyclic compounds of the general formula VIa or VIIa, respectively optionally in the presence of an acid binding agent at -10 to +100 °C, preferably 0 to 40 °C. The thus obtained compounds can be isolated as disclosed above.

The active ingredients according to the invention and the preparation of the microbicidal agents containing the same is shown in the following Examples.

Example 1

N-/2,6-dimethylphenyl/-glyoxylic acid amide diacetate

To a mixture of 11.63 g. /0.096 mole/ of 2,6-dimethyl aniline, 0.1 mole of pyridine and 80 ml. of toluene 19.45 g. /0.1 mole/ of glyoxylic acid chloride diacetate

/diacetoxy acetyl chloride, $n_D^{20}$=1.4276/ are added dropwise under stirring at 10 °C. The reaction is slightly exothermic. When the addition is completed the mixture is stirred for 2 hours at room temperature. The precipitated pyridine hydrochloride is filtered by suction and washed with some toluene. The filtrate is washed subsequently with water, diluted hydrochloric acid solution, diluted alkali and again with water. The organic layer is dried with sodium sulphate and filtered. The filtrate is concentrated in vacuo. The precipitated product is filtered by suction and washed with n-hexane. Yield: 19.1 g /72.3 %/, melting point: 105 °C.

Intensive peaks in the IR spectrum /KBr/: 3250, 1780, 1760, 1690, 1205, 1070, 1065, 790 $cm^{-1}$.

Example 2

N-Isopropyl-N-phenyl-glyoxylic acid amide diacetate

To a solution of 13.52 g. /0.1 mole/ N-isopropylaniline and 100 ml. of abs. benzene 29.17 g. /0.15 mole/ glyoxylic acid chloride diacetate are added dropwise under stirring at 15 °C followed by the solution of 11.0 g. of triethyl amine and 15 ml. of benzene at 15 °C. When the addition is completed the mixture is stirred for a further 2 hours. at room temperature. The mixture is subsequently washed with water, diluted hydrochloric acid solution, diluted alkali solution and again with water. The organic layer is dried above sodium sulphate. Benzene is distilled off after filtration. The residue is taken up in n-hexane and

the crystals are filtered by suction and recrystallized from iso-propylalcohol /charcoal/.

23.2 g. /79.2 %/ of the title product are obtained, m.p.: 79 °C

IR, $^{1}$H-NMR and mass spectra of the product correspond to the expected structure.

Example 3

Methyl ester of N-/2,6-dimethylphenyl/-N-/2,2-diacetoxy-acetyl/-alanine

To a solution of 38.90 g. /0.2 mole/ of glyoxylic acid chloride diacetate /2,2-diacetoxy-acetyl-chloride/ in 200 ml. of dry benzene 24.2 g. /0.116 mole/ of methyl ester of N-/2,6-dimethyl-phenyl/-alanine are added dropwise under stirring at 15 to 20 °C and under the same conditions a solution of 30.4 ml. of triethyl amine and 30 ml. of benzene is added. When the addition is completed the mixture is stirred for another 2 hours at room temperature. The mixture is subsequently washed with water, diluted hydrochloric acid, diluted alkali and again with water. The benzene layer is dried above sodium sulphate. After filtration benzene is distilled off in vacuo. The viscous substance is taken up in isopropyl alcohol. A crystalline slurry is obtained. The product is dissolved by heating /activ charcoal/ and filtered. After crystallization the product is filtered by suction. White crystalline product is obtained. Yield: 25.75 g. /60.8 %/, m.p.: 129 °C.

IR spectrum /KBr/: 3000, 1780, 1760, 1695, 1220,

0135934

1205, 800 $cm^{-1}$.

$^1H$-NMR, /TMS/: 1.1 /d, $CH_3$, -methyl/; 1.95 /s, $CH_3$-CO-/; 2.0 /s, $CH_3$-CO-/; 2.3 /s, 2-$CH_3$/; 2.5 /s, 6-$CH_3$/; 3.85 /s, $OCH_3$/; 4.50 /m, =N-CH-C=O/; 6.45 /s, -CO-CH=/; 7.15 /m, 3 aromatic H/.

Example 4

One may proceed as disclosed in Example 3 by using the same amounts and the methyl ester of N-/2,6-dimethyl--phenyl/ alanine is dissolved in 200 ml. of benzene. After stirring at 15 to 20 °C 0.2 mole of glyoxylic acid diacetate and a solution of 30.4 ml. of triethyl amine in 30 ml. of benzene are added. The mixture is stirred for another 2 hours and poured into 200 ml. of a 5 % NaOH solution. After separation the mixture is washed with 3 x 40 ml. of water and dried above $Na_2SO_4$. Benzene is distilled off in vacuo. The residue is recrystallized from isopropyl alcohol. Yield: 33.85 g /79.8 %/ of methyl ester of N-/2,6-dimethyl-phenyl-N-/2,2-diacetoxy-acetyl/-alanine, m.p.: 129-131 °C.

Example 5

N-/2-ethyl-6-methyl-phenyl/-N-methoxymethyl-2,2--diacetoxy-acetamide

To a 50 % xylene solution of 14.7 g. /0.1 mole/ 2--ethyl-6-methyl-phenyl-azomethine /Ib. $R^1$=$CH_3$, $R^2$=/-$C_2H_5$, $R^3$, $R^4$=H/ a solution of 22.0 g. /0.11 mole/ 2,2-diacetoxy--acetyl chloride and 10 ml. of xylene are added dropwise under stirring at 35-40 °C. When the addition is completed

the mixture is stirred for another hour. The obtained intermediate chloromethyl compound is then added dropwise to 50 ml. of methanol at 30-40 °C. The mixture is allowed to stand overnight and the excess methanol and xylene are distilled in vacuo. The residue is taken up in ethyl acetate and washed with water, diluted alkali, diluted hydrochlorid acid and again with water and the organic layer is separated. The organic layer is dried above $Na_2SO_4$ and evaporated in vacuo. 10.85 g. of viscous product are obtained.

Example 6

N-/2-ethyl-6-methyl-phenyl/-N-/1,2,4-triazol-1--yl-methyl/-2,2-diacetoxy-acetamide

To a 50 % solution of 14.7 g. /0.1 mole/ of 2-ethyl--6-methyl-phenyl-azomethine in xylene a solution of 22.0 g. /0.11 mole/ of 2,2-diacetoxy-acetyl chloride and 10 ml. of xylene are added dropwise at 35-40 °C under stirring. When the addition is completed the mixture is stirred for another hour. To the obtained N-/2-ethyl-6-methyl-phenyl/-N-chloromethyl-2,2-diacetoxy-acetamide solution 20 ml. of ethyl acetate are added whereafter the mixture is added under stirring to a mixture of 6.9 g. /0.1 mole/ triazole, 15 ml. ethyl acetate and 11 g. of triethyl amine. After the addition the mixture is stirred for another 2 hours and filtered. Triethyl amine hydrochloride is washed with some ehtyl acetate. The filtrate is washed with water, diluted hydrochloric acid, diluted NaOH and again with water. The organic

layer is dried above $Na_2SO_4$ and filtered. The volatile substances are distilled in vacuo. 21.42 g. of thick, oily product are obtained.

Example 7 to 22

The following compounds may be prepared by any of the methods disclosed above:

| Example | $R^1$ | $R^2$ | $R^3$ | R | $X^1$ | $X^2$ | m.p. °C |
|---|---|---|---|---|---|---|---|
| 7 | $CH_3$ | H | H | H | $COCH_3$ | $COCH_3$ | |
| 8 | $OCH_3$ | H | H | H | $COCH_3$ | $COCH_3$ | |
| 9 | Cl | H | H | H | $COCH_3$ | $COCH_3$ | 81-82 |
| 10 | $COOCH_3$ | H | H | H | $COCH_3$ | $COCH_3$ | |
| 11 | $C_2H_5$ | 6-$C_2H_5$ | H | H | $COCH_3$ | $COCH_3$ | 98-100 |
| 12 | $CH_3$ | 6-$C_2H_5$ | H | H | $COCH_3$ | $COCH_3$ | 82 |
| 13 | $CH_3$ | H | H | $CH/CH_3/COOCH_3$ | $COCH_3$ | $COCH_3$ | |
| 14 | $CH_3$ | 6-$C_2H_5$ | H | $CH/CH_3/COOCH_3$ | $COCH_3$ | $COCH_3$ | 101 |
| 15 | $C_2H_5$ | 6-$C_2H_5$ | H | $CH/CH_3/COOCH_3$ | $COCH_3$ | $COCH_3$ | 94-96 |
| 16 | $CH_3$ | 6-$CH_3$ | 4-$CH_3$ | $CH/CH_3/COOCH_3$ | $COCH_3$ | $COCH_3$ | |
| 17 | $CH_3$ | 6-$CH_3$ | H | $CH/CH_3/COOCH_3$ | $CH_3$ | $CH_3$ | |
| 18 | $CH_3$ | 6-$CH_3$ | H | $CH/CH_3/COOCH_3$ | $-CH_2-CH_2-$ | | |
| 19 | $CH_3$ | 6-$CH_3$ | H | pyrazol-1-yl--methyl | $COCH_3$ | $COCH_3$ | |
| 20 | $CH_3$ | 6-$CH_3$ | H | pyrazol-1-yl--methyl | $-CH_2-CH_2-$ | | |
| 21 | H | H | H | $-CH/CH_3/_2$ | $CH_3$ | $CH_3$ | |
| 22 | H | H | H | $-CH/CH_3/_2$ | $COCH_2Cl$ | $COCH_2Cl$ | |

Example 23

Water dispersible powder composition is prepared from the compound according to Example 3 by using excipients in the following ratio:

10.0 % by mass of the compound according to Example 3
3.0 % by mass Wettol NT-1 /Na-alkyl naphtaline-sulfonate/
5.0 % by mass Borrespoerse 3A /Na-lignine-sulfonate/
82.0 % by mass Wessalon S /precipitated $SiO_2$/.

A blend of the above materials is ground after homogenisation and rehomogenized.

The wet residue of the composition on a screen of 45 /um opening /CIPAC MT 59.3/ is less than 2 %, wetting time 38 sec. /CIPAC MT 53.1.1./ Floatability 38 % /CIPAC MT 15.1/

/CIPAC= Collaborative International Pesticides Analytical Council Ltd. 1980/.

Example 24

Water dispersible powder composition is prepared from the compound according to Example 3 by using the following excipients:

50 % by mass of the compound according to Example 3
5.0 % by mass of Wessalon S
3.0 % by mass of Wettol NT-1
5.0 % by mass of Borrespoerse 3A
0.5 % by mass of Mowiol 4-88 /poly vnyl alcohol/
36.5 % by mass of kieselgel F-195 /ground diatomaeous-earth/

0135934

The wet screen residue of the composition on a screen of 45 /um opening is smaller than 0.9 % /CIPAC MT. 59.3/. Wetting time: 50 sec /CIPAC MT. 53.3.1./ floatability 87 % /CIPAC MT. 15.1/.

Example 25

From the compounds according to the invention a 10 % wettable powder composition is prepared according to Example 23 and then 100, 500, 1000 and 2000 ppm aqueous suspensions are prepared by dilution with water. Tomato seedlings with 3 to 4 leaves were sprayed with the suspensions. When the spray liquid is dried the tomato seedlings are infected with Phytophtora infestans zoosporangium suspension. When the incubation period is over the infection grade is evaluated on the 6th day after the infection on a scale 0 to 4. The evaluation is performed as follows:

0 = no infection

1 = 1-30 % infection

2 = 31-60 % infection

3 = 51-90 % infection

4 = 91-100 % infection

The following results are obtained:

| Compound | Concentration ppm | | Extent of infections as an average value of repeated tests | |
|---|---|---|---|---|
| 13 | 100 | 500 | 1000 | 2000 |
| | 0.8 | 0.0 | 0.0 | 0.0 |
| 10 | 100 | 500 | 1000 | 2000 |
| | 1.0 | 0.0 | 0.0 | 0.0 |
| 3 | 100 | 500 | 1000 | 2000 |
| | 0.0 | 0.0 | 0.0 | 0.0 |
| 2 | 100 | 500 | 1000 | 2000 |
| | 0.6 | 0.0 | 0.0 | 0.0 |
| Infected control | 4 | - | - | - |

The new glyoxanilides can particularly be used as plant fungicides against fungi belonging to the class of Phycomycetes. Some of the compounds show strong phytotoxicity and bactericidal activity in the course of the biological tests.

0135934

# Claims

1. Glyoxanilide derivatives of the general formula

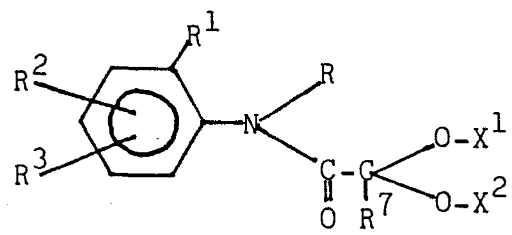

I

wherein

R stands for H, $C_1$-$C_4$ alkyl, $C_{1-4}$ alkoxy $C_{1-4}$ alkyl, $C_2$-$C_5$ alkenyl, allyl, $C_{2-5}$ alkinyl, halo-methyl, $-CH/R^4/COR^5$, $-CH/R^4/CN$, $-CHR^4/CHR^6/_nCOOR^5$, a heterocyclic group optionally substituted by one or more groups selected from alkyl and halogen having the general formula

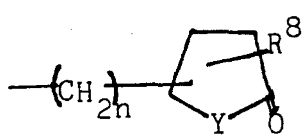

V

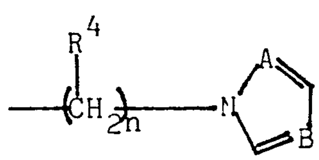

VI

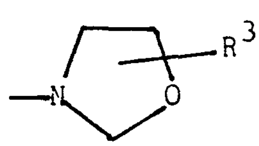

VII

or benzyl group

wherein the phenyl group is optionally substituted by one or more alkyl, alkoxy, halogen, haloalkyl, nitro, nitrile or $-CH_2SCN$, $-CH_2-Y-P{=}Z/OR^{12}/\ /OR^{13}/$ or an acetal group of the general formula

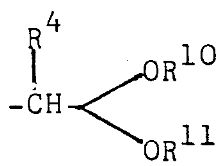

VIII

$R^1$ stands for H, $C_1$-$C_4$ alkyl, $C_1$-$C_4$ alkoxy, haloalkyl, $-COOR^{14}$,

$R^2$ stands for H, $C_1$-$C_4$ alkyl, $C_1$-$C_4$ alkoxy, halogen, haloalkyl, CN, SCN,

$R^3$ stands for H, halogen, $-CH_3$

$R^4$ represents H, $C_1$-$C_4$ alkyl

$R^5$ represents $C_1$-$C_4$ alkoxy, $-NR^{14}R^{15}$, OM

$R^6$ stands for $C_1$-$C_4$ alkyl

$R^7$ is H or $-CH_3$

$R^8$, $R^9$ represent H or $-CH_3$

$R^{10}$, $R^{11}$ stand for H, $C_1$-$C_4$ alkyl or together form a $C_1$-$C_4$ alkylene group

$R^{12}$, $R^{13}$ is H, $-CH_3$, $-C_2H_5$

$R^{14}$, $R^{15}$ represent H, $C_1$-$C_4$ alkyl or together form a $C_{4-5}$ alkylene group

$X^1$, $X^2$ stand for H, $-COR^{16}$, $C_1$-$C_4$ alkyl or together form the ethylene bridge of the 1,3-dioxolane ring

$R^{16}$ is H, $-CH_3$, $-CH_2Cl$, $-C_2H_5$

0135934

n is 0, 1, 2

Z, Y are O or S

A, B stand for -CH=, -N=

halo stands for F, Cl, Br

M is an equivalent of the following cations:

Na, K, $NH_4^+$, Mg, Ca, Zn, Cu, Mn, Fe.

2. Microbicidal composition comprising an active ingredient of the general formula I as claimed in claim 1.

3. A process for the preparation of compounds of the general formula I, which c o m p r i s e s

a/ reacting anilines of the general formula

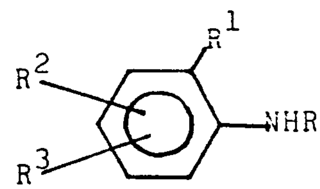

Ia

- wherein the substituents are as given above - with reactive derivatives, such as halides or anhydrides of glyoxylic acid acylates or acetals of the general formula

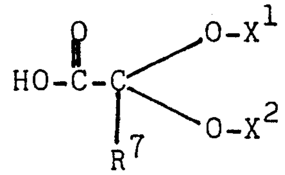

IX

in a solvent and optionally in the presence of an acid

binding agent at -10 - +120 °C, and isolating the desired product from the reaction mixture, or

b/ reacting azomethines of the general formula

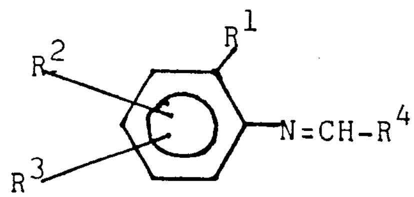

Ib

- wherein the substituents are as given above - with acid halides of glyoxylic acid acylate or acetal of the general formula IX in a solvent at -10 - +100 °C and reacting the obtained intermediate product of the general formula

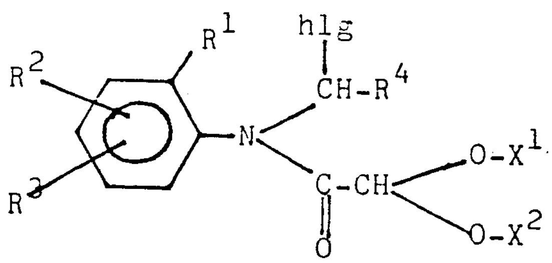

Ic

without isolation with a $C_1$-$C_4$ alcohol or one of the compounds of the general formula

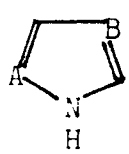

VIa

or

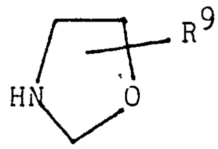

VIIa

optionally in the presence of an acid binding agent and isolating the desired product from the reaction mixture.